# FASCICULE DE BREVET EUROPEEN

(11) **EP 1 395 538 B1**
(45) Date de publication et mention de la délivrance du brevet: **30.12.2009**
(21) Numéro de dépôt: 02735556.9
(22) Date de dépôt: 22.05.2002
(51) Int. Cl.: C07C 33/28, C07C 33/48, C07C 43/23, A61K 31/047, A61K 31/085, A61K 8/34, A61Q 5/00, A61Q 19/00, A61P 17/00, A61P 35/00, A61P 19/00

(54) **ANALOGUES DE LA VITAMINE D**
VITAMIN-D-ANALOGA
VITAMIN D ANALOGUES

(30) Priorité: 22.05.2001 FR 0106731
(43) Date de publication de la demande: 10.03.2004
(73) Titulaire: GALDERMA RESEARCH & DEVELOPMENT, 06410 Biot (FR)
(72) Inventeur: BERNARDON, Jean-Michel, F-06000 Nice (FR)
(74) Mandataire: Allab, Myriam
(86) Numéro de dépôt international: PCT/FR2002/001726
(87) Numéro de publication internationale: WO 2002/094754

(56) Documents cités:
- WO-A-00/26167

## Description

L'invention concerne, à titre de produits industriels nouveaux et utiles, des composés bi-aromatiques analogues de la vitamine D.

L'invention concerne également leur procédé de préparation et leur utilisation dans des compositions pharmaceutiques destinées à un usage en médecine humaine ou vétérinaire, ou bien encore dans des compositions cosmétiques.

Les composés selon l'invention ont une activité marquée dans les domaines de la prolifération et de la différenciation cellulaire et trouvent des applications plus particulièrement dans le traitement topique et systémique des affections dermatologiques (ou autres) liées à un désordre de la kératinisation, des affections à composante inflammatoire et/ou immunoallergique et de l'hyperprolifération des tissus d'origine ectodermique (peau, épithélium...), qu'elle soit bénigne ou maligne. Ces composés peuvent en outre être utilisés pour lutter contre le vieillissement de la peau, qu'il soit photoinduit ou chronologique et traiter les troubles de la cicatrisation.

On peut également utiliser les composés selon l'invention dans des compositions cosmétiques pour l'hygiène corporelle et capillaire.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. Mais, il est maintenant admis que ses fonctions s'étendent bien au delà de la régulation du métabolisme osseux et de l'homéostasie calcique. Parmi celles-ci, peuvent être citées ses actions sur la prolifération et sur la différenciation cellulaire et le contrôle des défenses immunitaires. Leur découverte a ouvert la voie à de nouvelles approches thérapeutiques en dermatologie, cancérologie, ainsi que dans le domaine des maladies auto-immunes et celui des transplantations d'organes ou de tissus.
Un apport thérapeutique efficace s'est longtemps heurté à la toxicité de cette vitamine (hypercalcémie parfois mortelle). Actuellement, des analogues structuraux de la vitamine D sont synthétisés, dont certains ne conservent que les propriétés différenciatrices et n'ont pas d'action sur le métabolisme calcique.

La demanderesse a déjà proposé dans la demande WO 00/26167 (D1) de nouveaux composés analogues de la vitamine D qui montrent une activité sélective sur la prolifération et sur la différenciation cellulaire sans présenter de caractère hypercalcémiant. Ces composés analogues de la vitamine D sont en particulier plus facilement synthétisables et donc plus économiques par rapport à ce qui était connu antérieurement.

La Demanderesse vient de découvrir, de manière surprenante, que certains composés non spécifiquement décrits dans la demande WO 00/26167 (D1) mais vérifiant la formule générale décrite montrent une activité biologique très supérieure à celle des composés spécifiquement décrits. Cette activité est si forte qu'elle approche l'activité de la Vitamine D naturelle.

Ainsi, la présenté invention concerne au moins un composé choisi parmi les composés suivants:
- (4E,6E)-7-{3-[2-(3,4-Bis-hydroxymethyl-phenyl)-ethyl]-phenyl}-3-ethyl-nona-4,6-dien-3-ol,
- (E)-6-[3-(3,4-Bis-hydroxymethyl-benzyloxy)-phenyl]-1,1,1-trifluoro-2-trifluoromethyl-oct-5-en-3-yn-2-ol,
- (3E,5E)-6-[3-(3,4-Bis-hydroxymethyl-benzyloxy)-phenyl]-1,1,1-trifluoro-2-trifluoromethyl-octa-3,5-dien-2-ol,
- (E)-6-{3-[2-(3,4-Bis-hydroxymethyl-phenyl)-ethyl]-phenyl}-1,1,1-trifluoro-2-trifluoromethyl-oct-5-en-3-yn-2-ol,
- (3E,5E)-6-{3-[2-(3,4-Bis-hydroxymethyl-phenyl)-ethyl]-phenyl-1,1,1-trifluoro-2-trifluoromethyl-octa-3,5-dien-2-ol,
ainsi que leurs isomères géométriques et les composés pour lesquels une ou plusieurs des fonctions hydroxyles sont protégées par un groupement protecteur de type -(C=O)-R,
avec R représentant un radical alkyle linéaire ou ramifié ayant de 1 à 6 atomes de carbone, ou un radical aryle ayant de 6 à 10 atomes de carbone, ou un radical aralkyle ayant de 7 à 11 atomes de carbone, le radical aryle ou le radical aralkyle pouvant être mono ou disubstitué par un groupement hydroxy, par un groupement alkoxy ayant de 1 à 3 atomes de carbone, par un atome d'halogène, par une fonction nitro ou par une fonction amino, et leurs mélanges.

Par radical alkyle linéaire ou ramifié ayant de 1 à 6 atomes de carbone on entend un radical méthyle, éthyle, isopropyle, tertiobutyle ou hexyle.

Par radical aryle ayant de 6 à 10 atomes de carbone on entend un radical phényle ou naphtyle.

Par radical aralkyle ayant de 7 à 11 atomes de carbone on entend un radical benzyle ou méthyl-naphtyle.

Par atome d'halogène on entend un atome de fluor, brome ou chlore.

La présente invention a également pour objet les procédés de préparation des composés indiqués ci-dessus.

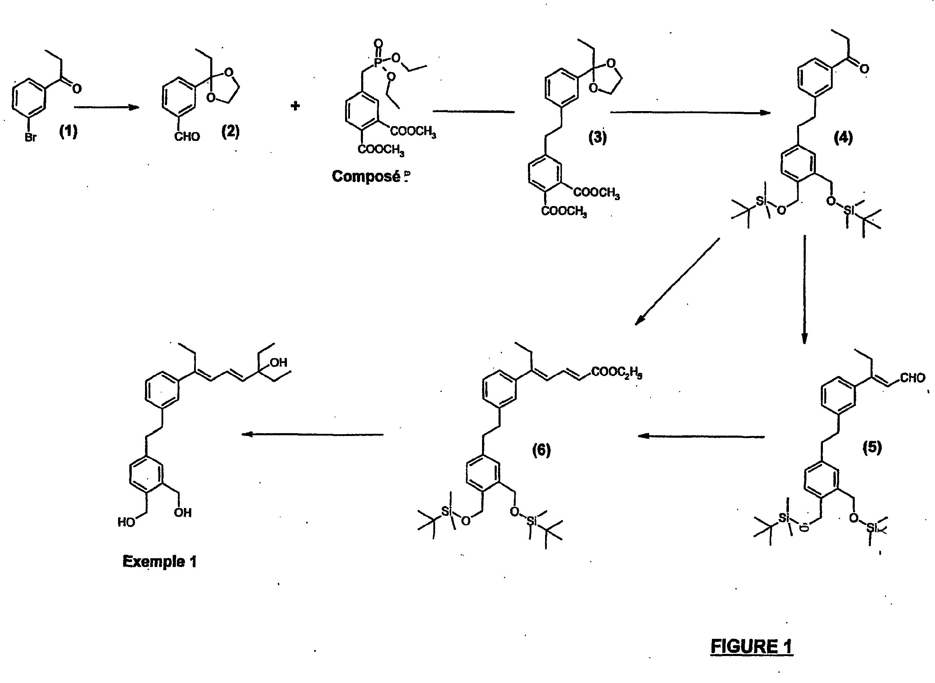

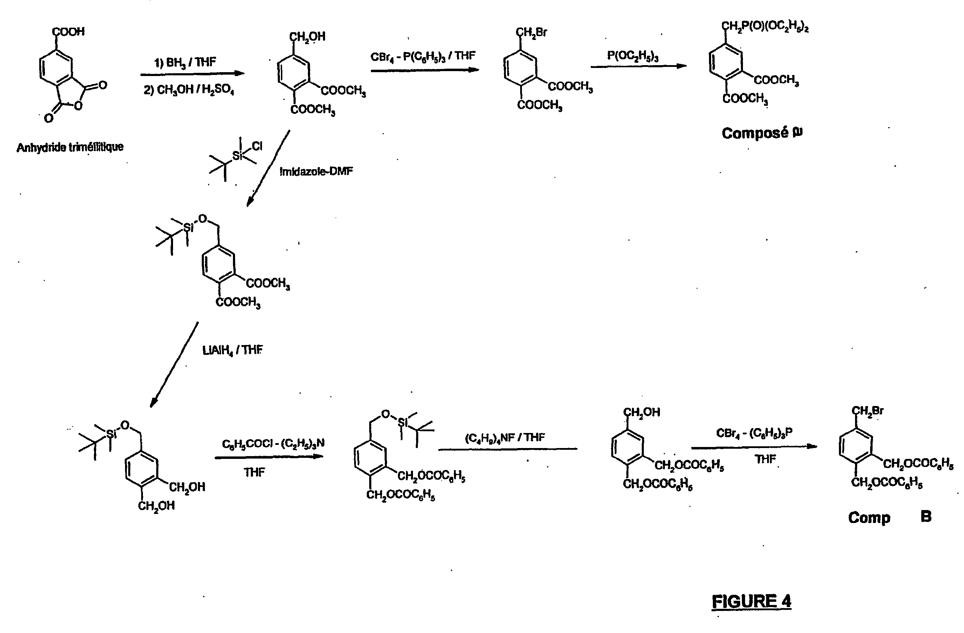

Les figures 1 à 4 représentent des schémas réactionnels qui peuvent être mis en oeuvre pour la préparation des composés selon l'invention.
Ainsi le composé de l'exemple 1 peut être obtenu (Figure 1) à partir du dérivé (6) par réaction avec l'ethyllithium à -78°C dans un solvant tel le THF puis déprotection des groupements hydroxyles en présence de fluorure de tétrabutyl ammonium.
Le composé (6) peut être obtenu à partir de la 3-bromopropiophénone (1) par une suite de réaction comprenant:
- la formation du dérivé (2) par protection de la fonction cétonique sous forme de dioxolanne puis formation de la fonction aldéhydique à partir du lithien correspondant en présence de DMF.
- La formation du dérivé (3) par une réaction de type Homer-Emmons entre le dérivé phosphonate (composé A) et le benzaldéhyde (2), puis hydogénation en présence de palladium sur charbon.
- La formation du dérivé (4) par réduction des fonctions esters en présence d'hydrure double de lithium et d'aluminium, déprotection de la cétone en présence d'acide paratoluènesulfonique et protection des fonctions alcools sous forme de *tert-*butyldimethylsilyl.
- La formation du dérivé (5) par une réaction de type Homer-Emmons avec le phosphonoacétate de triéthyle puis réduction de la fonction ester en présence d'hydrure double de lithium et d'aluminium et oxydation de la fonction alcool en présence de dioxyde de manganèse.
- La formation du dérivé (6) par une réaction de type Horner-Emmons entre le phosphonoacétate de triéthyle et le dérivé aldéhydique (5) ou directement par une réaction de type Homer-Emmons entre le phosphonocrotonate de triéthyle et le dérivé cétonique (4).
Le composé A pouvant être préparé à partir de l'anhydride triméllitique selon le schéma réactionnel représenté Figure 4.

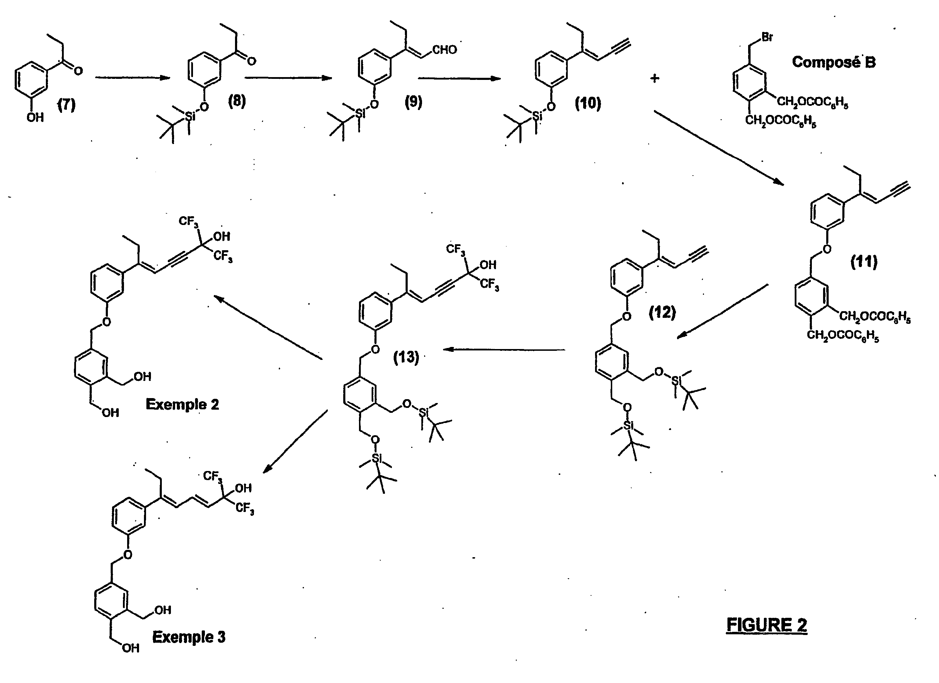

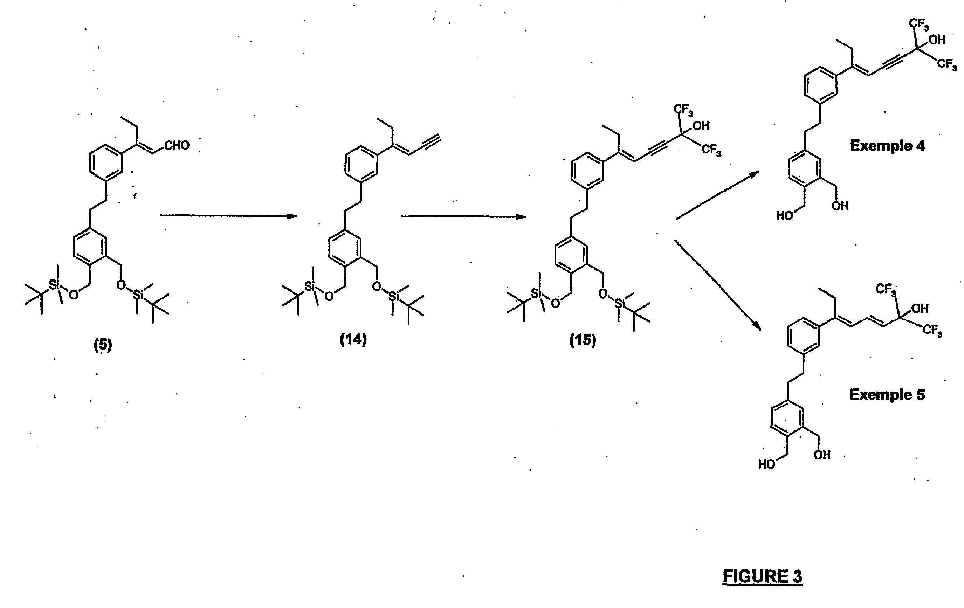

Les composés des exemples 2 et 4 peuvent être obtenus (Figure 2 et 3) respectivement à partir du dérivé (13) et du dérivé (15) par déprotection des fonctions alcools en présence de fluorure de tétrabutylammonium.
Les composés des exemples 3 et 5 peuvent être obtenus (Figure 2 et 3) respectivement à partir du dérivé (13) et du dérivé (15) par réduction de la triple liaison en double liaison de configuration *trans* avec de l'hydrure double de lithium et d'aluminium en présence de méthylate de sodium dans un solvant tel le THF puis déprotection des fonctions alcools en présence de fluorure de tétrabutylammonium.
Le composé (13) pouvant être obtenu à partir de la 3-hydroxypropiophenone (7) par une suite de réactions comprenant:
- La formation du dérivé (8) par protection du groupement phénol sous forme de *tert-*butyldiméthylsilyl.
- La formation du dérivé (9) par une réaction de type Homer-Emmons avec le phosphonoacétate de triéthyle puis réduction de la fonction ester en présence d'hydrure double de lithium et d'aluminium et oxydation de la fonction alcool en présence de dioxyde de manganèse.
- La formation du dérivé (10) par une réaction de type Corey-Fuchs.
- La formation du dérivé (11) par déprotection de la fonction phénolique puis réaction de couplage avec le dérivé bromé (composé B) en présence d'hydrure de sodium dans un solvant tel le DMF.
- La formation du dérivé (12) par déprotection des groupements benzoates puis reprotection sous forme de groupements tert-butyldiméthylsilyloxy.
- La formation du dérivé (13) par réaction avec le butyl lithium puis avec l'hexafluoroacétone.

Le composé B pouvant être préparé à partir de l'anhydride triméllitique selon le schéma réactionnel représenté Figure 4.

Le composé (15) pouvant être obtenu à partir du dérivé (5) par transformation de la fonction aldéhydique en fonction acétylénique (14) selon une réaction de type Corey-Fuchs puis lithiation avec le butyllithium et réaction avec l'hexafluoroacétone.

La protection des fonctions hydroxy se fait par une méthode habituelle comme décrite dans la littérature, par exemple par réaction d'un chlorure d'acide de type RCOCl correspondant dans un solvant tel que le THF ou le Dichlorométhane en présence d'une base telle que la pyridine ou la triéthylamine.

Les composés selon l'invention présentent des propriétés biologiques analogues à celles de la vitamine D, notamment les propriétés de transactivation des éléments de réponse à la vitamine D (VDRE), telles qu'une activité agoniste vis-à-vis de récepteurs de la vitamine D ou de ses dérivés. Par vitamines D ou leurs dérivés on entend par exemple les dérivés de la vitamine D2 ou D3 et en particulier la 1,25-dihydroxyvitamine D3 (calcitriol).

Cette activité agoniste vis-à-vis de récepteurs de la vitamine D ou de ses dérivés peut être mise en évidence "in vitro" par des méthodes reconnues dans le domaine de l'étude de la transcription génique (Hansen et al., The Society For Investigative Dermatologie, vol. 1, No 1, April 1996).

A titre d'exemple, l'activité agoniste VDR peut être testée sur la lignée cellulaire HeLa, par cotransfection d'un vecteur d'expression du récepteur VDR humain et du plasmide rapporteur p240Hase-CAT. L'activité agoniste peut être caractérisée dans ce système de cotransfection, par la détermination de la dose nécessaire pour atteindre 50 % de l'activité maximale du produit (AC50). Le détail du protocole de ce test ainsi que les résultats obtenus avec les composés selon l'invention, sont décrits dans l'exemple 7 de la présente demande.

Les propriétés biologiques analogues à la vitamine D peuvent être également mesurées par la capacité du produit à inhiber la prolifération des kératinocytes humaines normaux (KHN en culture). Le produit est ajouté à des KHN cultivés dans des conditions favorisant l'état prolifératif. Le produit est laissé au contact des cellules pendant cinq jours. Le nombre de cellules prolifératives est mesuré par incorporation de Bromodeoxyuridine (BRdU) dans l'ADN. Le protocole de ce test ainsi que les résultats obtenus avec les composés selon l'invention, sont décrits dans l'exemple 8 de la présente demande.

Les propriétés biologiques analogues à la vitamine D peuvent être également mesurées par la capacité du produit à induire la différentiation des cellules de leucémie promyélocytaire HL60. Le protocole de ce test ainsi que les résultats obtenus avec les composés selon l'invention, sont décrits dans l'exemple 9 de la présente demande.

L'activité agoniste aux récepteurs de la vitamine D des composés de l'invention peut être également évaluée "in vivo" par induction de la 24-Hydroxylase chez la souris SKH. (Voorhees and al. 1997.108 : 513-5 18). Le protocole de test utilisé ainsi que les résultats obtenus avec les composés selon l'invention, sont décrits dans l'exemple 10 de la présente demande.

La présente invention a également pour objet à titre de médicament les composés décrits ci-dessus.

Les composés selon l'invention conviennent particulièrement bien dans les domaines de traitement suivants :
- des affections dermatologiques liées à un désordre de la kératinisation portant sur la différenciation et sur la prolifération telles que les acnés vulgaires, comédoniennes, polymorphes, rosacées, les acnés nodulokystiques, conglobata, les acnés séniles, les acnés secondaires telles que l'acné solaire, médicamenteuse ou professionnelle ;
- des ichtyoses, des états ichtyosiformes, de la maladie de Damier, des kératodennies palmoplantaires, des leucoplasies et des états leucoplasiformes, du lichen cutané ou muqueux (buccal) ;
- des affections dermatologiques avec une composante immuno-allergique inflammatoire, avec ou sans trouble de la prolifération cellulaire, et telles que toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
- des proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que les lésions précancéreuses cutanées telles que les kératoacanthomes;
- des dermatoses immunes telles le lupus érythémateux, les maladies immunes bulleuses ou les maladies du collagène, telle la sclérodermie;
- des affections dermatologiques ou générales à composante immunologique;
- des troubles de la fonction sébacée tels que l'hyperséborrhée de l'acné ou la séborrhée simple;
- des désordres cutanés dus à une exposition aux rayonnements U.V., du vieillissement de la peau, qu'il soit photo-induit ou chronologique, des pigmentations et les kératoses actiniques, ou toutes pathologies associées au vieillissement chronologique ou actinique;
- des troubles de la cicatrisation ou des vergetures,
- des affections inflammatoires telles que l'arthrite, des affections d'origine virale au niveau cutané ou général, telle que le syndrome de Kaposis;
- des troubles ophtalmologiques, notamment les cornéopathies;
- des états cancéreux ou précancéreux de cancers présentant ou pouvant être induits par des récepteurs de vitamine D, tels que le cancer du sein, la leucémie, les syndromes myélodysplasiques et les lymphomes, les carcinomes des cellules de l'épithélium malpighien et les cancers gastro-intestinaux, les mélanomes, et l'ostéosarcome ;
- de l'alopécie de différentes origines, notamment l'alopécie due à la chimiothérapie ou aux rayonnements;
- des affections immunitaires, telles que les maladies auto-immunes, le diabète sucré de type 1, la sclérose en plaques, le lupus et les affections de type lupus, l'asthme, la glomérulonéphrite; des dysfonctionnements sélectifs du système immunitaire, telles que le SIDA, du rejet immun;
- des affections endocriniennes ;
- des affections caractérisées par une gestion anormale du calcium intracellulaire, des pathologies dans lesquelles le métabolisme calcique est impliqué, telle que l'ischémie musculaire;
- des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, ou les troubles de la fonction parathyroïdienne;
- des affections du système cardiovasculaire telles que l'artériosclérose ou l'hypertension ainsi que le diabète non-insulino dépendant.

Dans les domaines thérapeutiques mentionnés ci-dessus, les composés selon l'invention peuvent être avantageusement employés en combinaison avec des rétinoïdes, avec des corticostéroïdes ou des oestrogènes, en association avec des anti-oxydants, avec des α-hydroxy acides, des β-hydroxy acides ou α-céto acides ou leurs dérivés, avec des bloqueurs de canaux ioniques, ou encore en association avec d'autres médicaments connus pour interférer avec le système immunitaire (par exemple la cyclosporine, le FK 506, les glucocorticoïdes, les anticorps monoclonaux, les cytokines ou les facteurs de croissance...).

Par rétinoides, on entend des ligands des récepteurs RAR ou RXR, naturels ou synthétiques.

Par anti-oxydants on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique, ainsi que leurs sels, amides ou esters.

Par β-hydroxyacides ou leurs dérivés, on entend par exemple l'acide salicylique ainsi que ses sels, amides ou esters.

Par bloqueurs de canaux ioniques on entend par exemple les bloqueurs de canaux potassiques, et en particulier le Minoxidil (2,4-diamino-6-pipéridino-pyrimidine-3-oxyde) et ses dérivés.

La présente invention a également pour objet une composition pharmaceutique comprenant au moins un composé tel que défini ci-dessus.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection. Les composés selon l'invention sont généralement administrés à une dose journalière d'environ 0,001 µg/kg à 1000 µg/kg et de préférence d'environ 0,01 µg/kg à 100 µg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé selon l'invention à une concentration de préférence comprise entre 0,0001 et 5 % et de préférence entre 0,001 à 1 % par rapport au poids total de la composition.

Les composés selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec les rétinoïdes, avec des corticostéroïdes, en association avec des anti-oxydants, avec des α-hydroxy ou α-céto acides ou leurs dérivés, ou encore avec des bloqueurs de canaux ioniques.

Les différents produits pris en association avec les composés de la présente invention sont tels que définis ci-dessus.

La présente invention vise donc également une composition cosmétique contenant, dans un support cosmétiquement acceptable, au moins un composé tel que défini ci-dessus. Cette composition cosmétique peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé selon l'invention dans les compositions cosmétiques peut être comprise entre 0,001 et 3 % en poids par rapport au poids total de la composition.

Les compositions pharmaceutiques et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chlore-3-méthyl-1,2,4-benzothiadiazine1,1-dioxyde) et le Phénytoïn (5,4-diphényl-imidazolidine-2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11 -trynoïque, leurs esters et les amides.

Les compositions selon l'invention peuvent également contenir des agents d'amélioration de la saveur, des agents conservateurs tels que les esters de l'acide parahydroxybenzoïque, les agents stabilisateurs, des agents régulateurs d'humidité, des agents régulateurs de pH, des agents modificateurs de pression osmotique, des agents émulsionnants, des filtres UV-A et UV-B, des ami-oxydants, tels que l'a-tocophérol, le butylhydroxyanisole ou le butylhydroxytoluène.

On va maintenant donner, à titre d'illustration et sans aucun caractère limitatif, les exemples d'obtention des composés selon l'invention, ainsi que diverses formulations concrètes à base de tels composés ainsi que les exemples des test d'évaluation de l'activité biologique des composés selon l'invention.

### EXEMPLE 1

### (4E,6E)-7-{3-[2-(3,4-Bis-hydroxymethylphenly)-ethyl]-phenyl}-3-ethyl-nona-4,6-dien-3-ol

a) 2-(3-Bromo-phenyl)-2-ethyl-[1,3]dioxolane
   15 g (70 mmol) de 3-bromo-propiophenone sont dissous dans 250 mL de toluene, et 20 mL (350 mmol) d'éthylène glycol sont ajoutés, ainsi que 660 mg (3,5 mmol) d'acide paratoluenesulfonique. Le montage est équipé d'un extracteur d'eau de type Dean-Stark, et le milieu réactionnel et porté à reflux pendant 20 h. Après refroidissement, traitement par une solution de bicarbonate de potassium et extraction avec de l'éther éthylique, le produit désiré est pur sans purification, et obtenu sous forme d'huile jaunâtre (m = 17,8 g ; R = 99%).
b) 3-(2-Ethyl-[1,3]dioxolan-2-yl)-benzaldehyde
   17,8 g (70 mmol) de 2-(3-bromo-phenyl)-2-ethyl-[1,3]dioxolane sont dissous dans 300 mL de THF et le mélange est refroidi à -78°C. 34 mL (85 mmol) d'une solution de butyllithium 2,5 M sont additionnés lentement, et le mélange est agité pendant 30 minutes. 8,1 mL (105 mmol) de DMF sont alors ajoutés, et le milieu est ramené à O°C, puis traité par une solution saturée de chlorure d'ammonium. Après extraction avec de l'éther éthylique, l'aldéhyde désiré est obtenu sous forme d'huile jaune ( m = 14 g ; R =97%).
c) 4-{(E)-2-[3-(2-Ethyl-[1,3]dioxolan-2-yl)-phenyll-vinyl)-phthalate de diméthyle
   10,3 g (30 mmol) de 4-(diethoxy-phosphorylmethyl)-phthalate de diméthyle sont dissous danse 100 mL de THF anhydre, et le mélange est refroidi à 0°C. 3,4 g (30 mmol) de tert-butylate de potassium sont additionnés par portions, et le milieu est agité pendant 30 minutes. Une solution de 5,6 g (27,3 mmol) de 3-(2-ethyl-[1,3]dioxolan-2-yl)-benzaldehyde dans 50 mL de THF est alors additionnée goutte à goutte, et le milieu est agité pendant 2 heures à 0°C. Après le traitement usuel, le résidu est purifie par chromatographie sur colonne de silice (éluant heptane 80 - acétate d'éthyle 20). Une huile jaune est obtenue ( m = 9,6 g ; R = 89%).
d) 4-{2-[3-(2-Ethyl-[1,3]dioxolan-2-yl)-phenyl]-ethyl}-phthalate de diméthyle
   9,5 g (24 mmol) de 4-{(E)-2-[3-(2-éthyl-[1,3]dioxolan-2-yl)-phenyll-vinyl}-phthalate de diméthyle sont dissous dans un mélange de 120 mL d'acétate d'éthyle et 5 mL de triéthylamine. Le milieu réactionnel est dégasé avec un flux d'azote pendant 10 minutes, puis 1 g de palladium sur charbon 5% est ajouté. Le milieu réactionnel est alors chauffé à 80°C tandis qu'une pression de 4 bars d'hydrogène est appliquée pendant 4 heures. Le milieu est alors filtré et concentré sous pression réduite. Le résidu est purifié par chromatographie sur colonne de silice. Une huile incolore est obtenue ( m = 7,5 g ; R = 80%).
e) (4-{2-[3-(2-Ethyl-[1,3]dioxolan-2-yl)-phenyl]-ethyl}-2-hydroxymethyl-phenyl)-methanol
   2,9 g (75 mmol) d'aluminohydrure de lithium sont suspendus dans 20 mL d'éther éthylique. Une solution de 7,5 g (19 mmol) de 4-{2-[3-(2-éthyl-[1,3]dioxolan-2-yl)-phenyl]-ethyl}-phthalate de diméthyle dans 100 mL d'éther éthylique est additionnée goutte à goutte. Après 20 minutes d'agitation à température ambiante, le milieu réactionnel est traité par addition de 15 mL d'eau, 1,5 mL de soude 15% et 4,5 mL d'eau, puis filtré et concentré sous pression réduite. Une huile incolore est obtenue ( m = 6,4 g ; R = 99 %).
f) 1-{3-[2-(3,4-Bis-hydroxymethyl-phenyl)-ethyl]-phenyl}-propan-1-one
   6,4 g (18,7 mmol) de (4-{2-[3-(2-éthyl-[1,3]dioxolan-2-yl)-phenyll-ethyl}-2-hydroxymethyl-phenyl)-methanol sont dissous dans un mélange de 40 mL d'eau et 40 mL d'acétone. 650 mg d'acide paratoluènesulfonique sont ajoutés, et le milieu est agité pendant 5 heures. Après le traitement usuel, le produit désiré est pur sans purification, et obtenu sous forme d'huile incolore ( m = 5,57 g ; R = 100 %).
g) 1-(3-{2-[3,4-Bis-(tert-butyl-dimethyl-silanyloxymethyl)-phenyl]-ethyl}-phenyl)-propan-1-one
   5,5 g (18,5 mmol) de 1-{3-[2-(3,4-bis-hydroxymethyl-phenyl)-ethyl]-phenyl}-propan-1-one sont dissous dans 50 mL de DMF anhydre, et le mélange est refroidi à 0°C. 6,7 g (45 mmol) de tert-butyldimethylchlorosilane et 3,5 g (52 mmol) d'imidazole sont ajoutés. Le milieu est ramené à température ambiante et agité pendant 2 h. Après traitement par une solution saturée de chlorure d'ammonium puis extraction avec de l'acétate d'éthyle, les phases organiques sont rassemblées, rincées avec de l'eau, séchées et concentrées sous pression réduite. Une huile incolore est obtenue ( m = 9,6 g ; R = 99 %).
h) (E)-3-(3-{2-[3,4-Bis-(*tert*-butyl-dimethyl-silanyloxymethyl)-phenyl]-ethyl}-phenyl)-pent-2-enoate d'éthyle
   11,1 mL (56 mmol) de phosphonoacétate de triéthyle sont dissous dans 100 mL de THF. 2,2 g (55 mmol) d'hydrure de sodium 60% sont alors ajoutés, et le milieu est agité pendant 30 minutes à température ambiante. Une solution de 9,5 g (18 mmol) de 1-(3-{2-[3,4-bis-(tert-butyl-dimethyl-silanyloxymethyl)-phenyll-ethyl}-phenyl)-propan-1-one dans 100 mL de THF est alors additionnée goutte à goutte. Le milieu est agité pendant 6 heures, puis traité avec de l'eau et extrait avec de l'acétate d'éthyle. Le résidu obtenu est purifié par chromatographie sur gel de silice (éluant acétate d'éthyle 10 - heptane 90). Une huile jaune est obtenue (m = 5,8 g ; R=56%).
i) (E)-3-(3-{2-[3,4-Bis-(*tert*-butyl-dimethyl-silanyloxymethyl)-phenyl]-ethyl}-phenyl)-pent-2-en-1-ol
   0,75 g (19 mmol) d'aluminohydrure de lithium sont suspendus dans 10 mL d'éther éthylique. Une solution de 5,6 g (9,6 mmol) de (E)-3-(3-{2-[3,4-bis-(*tert*-butyl-dimethyl-silanyloxymethyl)-phenyl]-ethyl}-phenyl)-pent-2-enoate d'éthyle dans 50 mL d'éther éthylique est additionnée goutte à goutte. Après 20 minutes d'agitation à température ambiante, le milieu réactionnel est traité par addition de 0,75 mL d'eau, 0,75 mL de soude 15% et 1,5 mL d'eau, puis filtré et concentré sous pression réduite. Une huile incolore est obtenue ( m = 5,26 g ; R = 99%).
j) (E)-3-(3-{2-[3,4-Bis-(tert-butyl-dimethyl-silanyloxymethyl)-phenyl]-ethyl}-phenyl)-pent-2-enal
   2,8 g (5 mmol) de (E)-3-(3-{2-[3,4-bis-(*tert*-butyl-dimethyl-silanyloxymethyl)-phenyl]-ethyl}-phenyl)-pent-2-en-1-ol sont dissous dans 50 mL de dichlorométhane. 4,3 g (50 mmol) de dioxyde de manganese sont additionnés, et le milieu réactionnel est agité pendant 12 heures, puis filtré et concentré sous pression réduite. Le produit désiré est obtenu sous forme d'huile jaune (m = 2,8 g ; R = 100 %).
k) (2E,4E)-5-(3-{2-[3,4-Bis-(*tert*-butyl-dimethyl-silanyloxymethyl)-phenyl]-ethyl)-phenyl)-hepta-2,4-dienoate d'éthyle
   1,3 mL (6,5 mmol) de phosphonoacétate de triéthyle sont dissous dans 20 mL de THF. 260 mg (6,5 mmol) d'hydrure de sodium 60% sont alors ajoutés, et le milieu est agité pendant 30 minutes à température ambiante. Une solution de 2,8 g (5 mmol) (E)-3-(3-{2-[3,4-bis-(*tert*-butyl-dimethyl-silanyloxymethyl)-phenyll-ethyl}-phenyl)-pent-2-enal dans 30 mL de THF est alors additionnée goutte à goutte. Le milieu est agité pendant 6 heures, puis traité avec de l'eau et extrait avec de l'acétate d'éthyle. Le résidu obtenu est purifié par chromatographie sur gel de silice (éluant acétate d'éthyle 10 - heptane 90). Une huile jaune est obtenue (m = 2,52 g ; R = 81 %).
l) (4E,6E)-7-{3-[2-(3,4-Bis-hydroxymethyl-phenyl)-ethyl]-phenyl}-3-ethyl-nona-4,6-dien-3-ol
   1,7 g (2,7 mmol) de (2E,4E)-5-(3-{2-[3,4-bis-(*tert*-butyl-dimethyl-silanyloxymethyl)-phenyl]-ethyl}-phenyl)-hepta-2,4-dienoate d'éthyle sont dissous dans 120 mL de THF anhydre, et le mélange est refroidi à -78°C. 13,5 mL (13,5 mmol) d'une solution d'ethyllithium (1,0 - 1,5 M) est additionnée, et le milieu est agité à cette température pendant 1 heure, puis ramenée à 0°C et traitée avec une solution saturée de chlorure d'ammonium. Le résidu obtenu est dissous dans 50 mL de THF puis 6,5 mL (6,5 mmol) d'une solution de fluorure de tetrabutylammonium est additionnée. Après 30 minutes d'agitation et le traitement usuel, le résidu obtenu est purifié par chromatographie sur colonne de silice. Le produit désiré est obtenu sous forme d'huile incolore ( m = 200 mg ; R = 18 %).
   RMN **¹H** (DMSO): 0,82 (t, 6H, J = 7,5 Hz); 0,89 (t, 3H, J = 7,4 Hz); 1,41-1,53 (m, 4H); 2,41 (q, 2H, J = 7,4 Hz); 2,83 (s, 4H); 4,01 (s, 1H); 4,41 (t, 2H, J = 8 Hz); 4,45 (t, 2H, J = 8 Hz); 4,94-4,98 (m, 2H); 6,31 (d, 1H, J = 15,0 Hz); 6,46 (d, 1H, J = 11,0 Hz); 7,08-7,29 (m, 7H); 7,42-7,45 (m, 1H).

### EXEMPLE 2

### (E)-6-[3-{3,4-Bis-hydroxymethyl-benzyloxy)phenyl]-1,1,1-trifluoro-2-trifluoromethyl-oct-5-en-3-yn-2-ol

a) 3-(*tert*-butyl-diméthyl-silanyloxy)-benzaldéhyde
   De manière analogue à l'exemple 1g, par réaction de 42,7 g (0,275 mol) de *tert*butyldiméthylchlorosilane avec 30,5 g (0,2 mol) de 3-hydroxybenzaldéhyde et (20,4 g, 0,3 mol) d'imidazole. Après purification sur colonne de silice (dichlorométhane 20 - heptane 80), une huile jaune est obtenue (m= 55,9 g ; R= 95%).
b) 1-[3-(*tert*-Butyl-dimethyl-silanyloxy)-phenyl]-propan-1-ol
   50 g (0,21 mol) de 3-(tert-butyl-diméthyl-silanyloxy)-benzaldéhyde sont dissous dans 500 mL d'éther éthylique et le mélange est refroidi à 0°C. 80 mL (0,24 mol) d'une solution de bromure d'éthylmagnésium 3,0 M sont additionnés lentement, et le mélange est agité pendant 5 heures. Après le traitement usuel, le résidu obtenu est purifié par chromatographie sur colonne de silice (eluant acétate d'éthyle 20 / heptane 80). Une huile incolore est obtenue (m = 45,8 g ; R = 82%).
c) 1-[3-(*tert*-Butyl-dimethyl-silanyloxy)-phenyl]-propan-1-one
   De manière analogue à l'exemple 1j, par réaction de 45 g (0,17 mol) de 1-[3-(tert-butyldimethyl-silanyloxy)-phenyll-propan-1-ol avec (148 g, 1,7 mol) de dioxyde de manganese. Une huile jaune est obtenue (m = 45 g, R = 100%).
d) (E)-3-[3-(*tert*-Butyl-dimethyl-silanyloxy)-phenyl]-pent-2-enoate d'éthyle
   22,5 mL (113 mmol) de phosphonoacétate de triéthyle sont dissous dans 100 mL de THF. 4,5 g (113 mmol) d'hydrure de sodium 60% sont alors ajoutés, et le milieu est agité pendant 30 minutes à température ambiante. Une solution de 20 g (75,6 mmol) de 3-(*ter*t-butyl-dimethylsilanyloxy)-propan-1 -one dans 100 mL de THF est alors additionnée goutte à goutte. Le milieu est agité pendant 6 heures, puis traité avec de l'eau et extrait avec de l'acétate d'éthyle. Le résidu obtenu est purifié par chromatographie sur gel de silice (éluant acétate d'éthyle 10 - heptane 90). Une huile jaune est obtenue (m = 7,6 g ; R = 30 %).
e) (E)-3-[3-(*tert*-Butyl-dimethyl-silanyloxy)-phenyl)-pent-2-en-1-ol
   De manière analogue à l'exemple 1e, par réaction de 7,6 g (23 mmol) de (E)-3-[3-(tert-butyl-dimethyl-silanyloxy)-phenyll-pent-2-enoate d'éthyle avec 1,05 g (25 mmol) d'aluminohydrure de lithium. Une huile incolore est obtenue (m = 6,7 g ; R = 100%).
f) (E)-3-[3-(tert-Butyl-dimethyl-silanyloxy)-phenyl)-pent-2-en-1-a1
   De manière analogue à l'exemple 1j, par réaction de 6,7 g (23 mmol) de (E)-3-[3-(tert-butyl-dimethyl-silanyloxy)-phenyl)-pent-2-en- 1-ol avec 10 g (115 mmol) de dioxyde de manganese. Une huile jaune est obtenue (m = 4,7 g ; R = 71%).
g) *tert*-Butyl-[3-((E)-4,4-dibromo-1 -ethyl-buta-1,3-dienyl)-phenoxy]-dimethyl-silane
   1,17 g (18 mmol) de poudre de zinc, 4,7 g (18 mmol) de triphenylphosphine et 5,9 g (18 mmol) de tetrabromure de carbone sont agités pendant 45 minutes dans 150 mL de dichlorométhane. Une solution de 2,6 g (9 mmol) (E)-3-[3-(tert-Butyl-dimethyl-silanyloxy)- phenyl)-pent-2-en-1-al dans 10 mL de dichlorométhane est additionnée goutte à goutte. Le milieu réactionnel est agité pendant 1 heure, puis extrait avec un mélange d'eau et de dichlorométhane. Le résidu est filtré sur une colonne de silice (éluant dichlorométhane). Une huile jaune est obtenue (m = 3,3 g ; R = 83%).
h) *tert*-Butyl-[3-((E)-1-ethyl-but-1-en-3-ynyl)-phenoxy]-dimethyl-silane
   3,2 g (7,2 mmol) de *tert*-butyl-[3-((E)-4,4-dibromo-1-ethyl-buta-1,3-dienyl)-phenoxy]-dimethyl-silane sont dissous dans 50 mL et le mélange est refroidi à -78°C. 5,7 mL (14,4 mmol) d'une solution de butyllithium 2,5 M sont additionnés et le milieu est agité pendant 2 heures, puis est traité par une solution saturée de chlorure d'ammonium. Le résidu est purifié par chromatographie sur colonne de silice. Une huile jaune est obtenue (m =1,0 g ; R = 49%).
i) 3-((E)-1-Ethyl-but-1-en-3-ynyl)-phenol
   1 g (3,5 mmol) de *tert*-butyl-[3-((E)-1-ethyl-but-1-en-3-ynyl)-phenoxy]-dimethyl-silane sont 15 dissous dans 50 mL de THF, et 3,8 mL (38 mmol) d'une solution de fluorure de tetrabutylammonium 1,0 M sont ajoutés goutte à goutte. Le milieu est agité pendant 30 minutes, puis traité par une solution saturée de chlorure d'ammonium et extrait avec de l'acétate d'éthyle. Une huile jaune est obtenue (m = 690 mg ; R =100 %).
j) Benzoate de 2-benzoyloxymethyl-5-[3-((E)-1-ethyl-but-1-en-3-ynyl)-phenoxymethyl]-benzyle
   610 mg (3,5 mmol) 3-((E)-1-éthyl-but-1-en-3-ynyl)-phenol sont dissous dans 80 mL de DMF.150mg (3,7 mmol) d'hydrure de sodium sont ajoutés, et le milieu est agité à température ambiante. 1,5 g (6,2 mmol) de benzoate de 2-benzoyloxymethyl-5-broniomethyl-benzyle sont alors ajoutés, et le milieu est agité pendant 2 heures. Après le traitement usuel et purification par chromatographie sur colonne de gel de silice, une huile incolore est obtenue (m = 1,66 g ; R=88%).
k) 1-(3,4-Bis-tert-butyldimethylsilyloxymethyl-benzyloxy)-3-((E)-1-ethyl-but-1-en-3-ynyl)-benzene
   1,6 g (3 mmol) de benzoate de 2-benzoyloxymethyl-5-[3-((E)-1-ethyl-but-1-en-3-ynyl)-phenoxymethyll-benzyle sont dissous dans 15 mL d'une solution de carbonate de potassium 2% dans du méthanol. Le milieu réactionnel est agité pendant 2 heures, puis traité par une solution saturée de chlorure d'ammonium, et extrait avec de l'acétate d'éthyle. Le résidu obtenu est dissous dans 20 mL de DMF anhydre, et 900 mg (6 mmol) de *tert*butyldiméthylchlorosilane et 450 mg (6,6 mmol) d'imidazole sont ajoutés. Le milieu est agité pendant 10 heures à température ambiante. Après le traitement usuel, le résidu obtenu est purifié par chromatographie sur colonne de silice (éluant heptane 85 - acétate d'éthyle 85). Une huile orange est obtenue (m = 1,16 g ; R = 70 %).
l) (E)-6-{3-[3,4-Bis-(*tert*-butyl-dimethyl-silanyloxymethyl)-benzyloxy]-phenyl}-1,1,1-trifluoro-2-trifluoromethyl-oct-5-en-3-yn-2-ol
   1,16 g (2,1 mmol) de 1-(3,4-bis-tert-butyldimethylsilyloxymethyl-benzyloxy)-3-((E)-1-ethylbut-1-en-3-ynyl)-benzene est dissous dans 30 mL de THF anhydre, et le mélange est refroidi à -78°C. 930 µL (2,3 mmol) d'une solution de butyllithium 2,5 M sont additionnés goutte à goutte, et le milieu est agité pendant 15 minutes. Un flux d'hexafluoroacétone (gaz) est passé dans la solution pendant 10 minutes, et le milieu réactionnel est traité par une solution saturée de chlorure d'ammonium. Le résidu obtenu est purifié par chromatographie sur colonne de silice (éluant heptane 80 - acétate d'éthyle 20). Une huile jaune est obtenue (m = 1,35 g ; R = 89 %).
m) (E)-6-[3-(3,4-Bis-hydroxymethyl-benzyloxy)-phenyl]-1,1,-trifluoro-2-trifluoromethyloct-5-en-3-yn-2-ol
   De manière analogue à l'exemple 2i, par réaction de 350 mg (0,5 mmol) de (E)-6-{3-[3,4-Bis-(*tert*-butyl-dimethyl-silanyloxymethyl)-benzyloxy]-phenyl}-1,1,1-trifluoro-2-trifluoromethyloct-5-en-3-yn-2-ol avec 1,2 mL (1,2 mmol) d'une solution de fluorure de tetrabutylammonium 1,0 M. Une huile incolore est obtenue (m = 2 10 mg ; R = 80 %).
   **RMN ¹H** (CDCl,): 1,05 (t, 3H, J = 7,4 Hz); 2,72 (q, 2H, J = 7,4 Hz); 4,74 (s, 4H); 5,05 (s,2H); 5,69 (s, 1H); 6,91-7,Ol (m, 3H); 7,24 (m, 1H); 7,37 (s, 2H); 7,42 (s, 1H).

### EXEMPLE 3

### (3E,5E)-6-[3-(3,4-Bis-hydroxymethyl-benzyloxy)-phenyl]-1,1,1-trifluoro-2 trifluoromethyl-octa-3,5-dien-2-ol

a) (3E,5E)-6-{3-[3,4-Bis-(*tert*-butyl-dimethyl-silanyloxymethyl)-benzyloxy]-phenyl}-1,1,1-trifluoro-2-trifluoromethyl-octa-3,5-dien-2-ol
   135 mg (3,55 mmol) d'aluminohydrure de lithium sont dissous dans 10 mL de THF anhydre.385 mg (7,1 mmol) de methylate de sodium sont ajoutés, et le mélange est agité à température ambiante pendant 10 minutes. Une solution de 860 mg (1,2 mmol) de (E)-6-{3-[3,4-bis-(tertbutyl-dimethyl-silanyloxymethyl)-benzyloxy]-phenyl}-1,1,1,1-trifluoro-2-trifluoromethyl-oct-5-en-3-yn-2-ol (décrit à l'exemple 21) dans 7 mL de THF est additionnée goutte à goutte. Le milieu est chauffé à reflux pendant 2 heures, puis traité par addition successive de 120 µL d'eau, 120 µL de NaOH 15% et 35 µL d'eau. Après filtration, une épaisse huile jaunâtre est obtenue (m = 650 mg ; R = 76 %).
b) (3E,5E)-6-[3-(3,4-Bis-hydroxymethyl-benzyloxy)-phenyl]-1,1,1-trifluoro-2-trifluoromethyl-octa-3,5-dien-2-ol
   De manière analogue à l'exemple 2m, par réaction de 640 mg (0,89 mmol) de (3E,5E)-6-{3-[3,4-bis-(tert-butyl-dimethyl-silanyloxymethyl)-benzyloxy]-phenyl}-1,1,1-trifluoro-2-trifluoromethyl-octa-3,5-dien-2-ol avec 2,1 mL (2,1 mmol) d'une solution de fluorure de tetrabutylammonium 1,0 M. Une huile incolore est obtenue (m = 260 mg ; R = 60 %).
   **RMN ¹H** (DMSO): 1,1O (t, 3H, J = 7,5 Hz); 2,65 (q, 2H, J = 7,4 Hz); 4,55 (t, 4H, J = 5,2 Hz); 5,08-5,17 (m, 4H); 6,05 (d, 1H, J = 15,1 Hz); 6,64 (d, 1H, J = 11,2 Hz); 6,96 (dd, 1H, JI =11,2 Hz, J2 = 2 Hz); 7,08-7,51 (m, 6H); 8,27 (s, 1H).

### EXEMPLE 4

### (E)-6-{3-[2-(3,4-Bis-hydroxymethyl-phenyl)-ethyl]-phenyl}-1,1,1-trifloro-2-trifluoromethyl-oct-5-en-3-yn-2-ol

a) (E)-1-(3-{2-[3,4-Bis-(tert-butyl-dimethyl-silanyloxymethyl)-phenyl]-ethyl}-phenyl)-4,4-dibromo-1-ethyl-buta-1,3-diene
   De manière analogue à l'exemple 2g, par réaction de 9 g (16,3 mmol) de (E)-3-(3-{2-[3,4-bis-(tert-butyl-dimethyl-silanyloxymethyl)-phenyl]-éthyl}-phenyl)-pent-2-enal (préparé à l'exemple 1j) avec 2,1 g (32,5 mmol) de poudre de zinc, 8,5 g (32,5 mmol) de triphenylphosphine et 108 g (32,5 mmol) de tetrabromure de carbone. Une huile jaune est obtenue (m = 11,3 g ; R = 98%).
b) (E)-1-(3-{2-[3,4-Bis-(tert-butyl-dimethyl-sil∼yloxymethyl)-phenyl]-ethyl}-phenyl)-1-ethyl-but-1-en-3-yne
   De manière analogue à l'exemple 2 h, par réaction de 11,3 g (15,9 mmol) de (F)-1-(3-{2-[3,4-bis-(tert-butyl-dimethyl-silanoxyloxymethyl)-phenyl]-ethyl}-phenyl)-4,4-dibromo-1-ethyl-buta-1,3-diene avec 128 mL (32 mmol) d'une solution de butyllithium 2,5 M. Une huile jaune est obtenue (m = 85 g ; R = 97%).
c) (E)-6-(3-{2-[3,4-Bis-(tert-butyl-dimethyl-silanyloxymethyl)-phenyl]-ethyl}-phenyl)-1,1,1-trifluoro-2-trifluoromethyl-oct-5-en-3-yn-2-ol
   De manière analogue à l'exemple 21, par réaction de 5 g (9,1 mmol) de (E)-1-(3-{2-[3,4-bis-(tert-butyl-dimethyl-silanyloxymethyl)-phenyl]-ethyl}-phenyl)-1-ethyl-but-1-en-3-yne avec 4 mL (10 mmol) d'une solution de butyllithium 2,5 M et un flux d'hexafluoroacetone. Le produit désiré est obtenu sous la forme d'une huile jaune (m = 3,7 g ; R = 57 %).
d) (E)-6-(3-[2-(3,4-Bis-hydroxymethyl-phenyl)-ethyl]-phenyl)-1,1,1-trifluoro-2-trifluoromethyl-oct-5-en-3-yn-2-ol
   De manière analogue à l'exemple 2m, par réaction de 1 g (1,4 mmol) de (E)-6-(3-{2-[3,4-bis-(*tert*-butyl-dimethyl-silanyloxymethyl)-phenyl]-ethyl)-phenyl)-1,1,1-trifluoro-2-trifluoromethyl-oct-5-en-3-yn-2-ol avec 3,3 mL (3,3 mmol) d'une solution de fluorure de tetrabutylammonium 1,0 M. Un solide jaunâtre est obtenu (PF = 123°C ; m = 570 mg ; R =84 %).
   **RMN ¹H** (CDCl₃): 1,05 (t, 3H, J = 7,5 Hz); 2,75 (q, 2H, J = 7,5 Hz); 2,92 (s, 4H); 4,69 (s,4H); 5,67 (s, 1H); 7,05-7,31 (m, 7H).

### EXEMPLE 5

### (3E,5E)-6-{3-[2-(3,4-Bis-hydroxymethyl-phenyl)-ethyl]-phenyl}-1,1,1-trifluoro-2-trifluoromethyl-octa-3,5-dien-2-ol

a) (3E,5E)-6-(3-{2-[3,4-Bis-(tert-butyl-dimethyl-silanyloxymethyl)-phenyl]-ethyl}-phenyl)-1,1,1-trifluoro-2-trifluoromethyl-octa-3,5-dien-2-ol
   De manière analogue à l'exemple 3a, par réaction de 2,5 g (3,5 mmol) de (E)-6-(3-(2-[3,4-bis-(tert-butyl-dimethyl-silanyloxymethyl)-phenyl]-ethyl}-phenyl)-1,1,1-trifluoro-2-trifluoromethyl-oct-5-en-3-yn-2-ol (décrit à l'exemple 4c) avec 400 mg (10,4 mmol) d'aluminohydrure de lithium et 1,13 g (21 mmol) de methylate de sodium. Une huile jaune est obtenue (m = 1,27 g ; R = 51 %).
b) (3E,5E)-6-{3-[2-(3,4-Bis-hydroxymethyl-phenyl)-ethyl]-phenyl}-1,1,1-trifluoro-2-trifluoromethyl-octa-3,5-dien-2-ol
   De manière analogue à l'exemple 2m, par réaction de 1,2 g (1,67 mmol) de (3E,5E)-6-(3-{2-[3,4-bis-(tert-butyl-dimethyl-silanyloxymethyl)-phenyl]-ethyl}-phenyl)-1,1,1-trifluoro-2-trifluoromethyl-octa-3,5-dien-2-ol avec 4 mL (4 mmol) d'une solution de fluorure de tetrabutylammonium 1,0 M. Un solide blanc est obtenu (PF = 104°C ; m = 715 mg ; R = 87 %).
   RMN ¹H (DMSO): 1,02 (t, 3H, J = 7,5 Hz); 2,66 (q, 2H, J = 7,5 Hz); 2,93 (s, 4H); 4,71 (s,2H); 4,72 (s, 2H); 5,79 (d, 1H, J = 15,0 Hz); 6,24 (d, 1H, J = 11,0 Hz); 7,08-7,30 (m, 8H).

### EXEMPLE 6 : EXEMPLES DE FORMULATION

### 1) VOIE ORALE

(a) On prépare la composition suivante sous la forme d'un comprimé de 0,2 g

| | |
|---|---|
| Composé de l'exemple 1 | 0,005g |
| Amidon prégélatinisé | 0,065 g |
| Cellulose microcristalline | 0,075 g |
| Lactose | 0,050 g |
| Stérate de magnésium | 0,005 g |

Pour le traitement de l'ichtyose, on administre à un individu adulte 1 à 3 comprimés par jour pendant 1 à 12 mois selon la gravité du cas traité.
(b) On prépare une suspension buvable, destinée à être conditionnée en ampoules de 5 ml

| | |
|---|---|
| Composé de l'exemple 2 | 0,050 mg |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 1 à 12 mois selon la gravité du cas traité.
(c) On prépare la formulation suivante destinée à être conditionnée en gélules :

| | |
|---|---|
| Composé de l'exemple 4 | 0,0001 mg |
| Amidon de maïs | 0,060 g |
| Lactose q.s.p | 0,300 g |

Les gélules utilisées sont constituées de gélatine, d'oxyde de titane et d'un conservateur.
Dans le traitement du psoriasis, on administre à un individu adulte, 1 gélule par jour pendant 1 à 12 mois.
(d) On prépare la formulation suivante destinée à être conditionnée en gélules :

| | |
|---|---|
| Composé de l'exemple 5 | 0,02mg |
| Cyclosporine | 0,050 g |
| Amidon de maïs | 0,060 g |
| Lactose q.s.p. | 0,300 g |

Les gélules utilisées sont constituées de gélatine, d'oxyde de titane et d'un conservateur.
Dans le traitement du psoriasis, on administré à un individu adulte, 1 gélule par jour pendant 1 à 12 mois.

### 2) VOIE TOPIQUE

(a) On prépare la crème Eau-dans l'Huile non ionique suivante :

| | |
|---|---|
| Composé de l'exemple 3 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 1 à 12 mois.
(b) On prépare un gel en réalisant la formulation suivante :

| | |
|---|---|
| Composé de l'exemple 1 | 0,001 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95") q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 2 | 0,030 g |
| Propylène glycol | 5,000 g |
| Butylhydroxytoluène | 0,100g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 2 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 3 | 0,500 g |
| Acide rétinoique | 0,020 g |
| Alcool cétylique | 4,000 g |
| Monostéarate de glycérol | 2,500 g |
| Parahydroxybenzoate de propyle | 0,075 g |
| Stéarate de PEG 50 | 2,500 g |
| Beurre de Karité | 9,200 g |
| Propylène glycol | 2,000 g |
| Parahydroxybenzoate de méthyle | 0,075 g |
| Eau déminéralisée stérile q.s.p. | 100,000g |

Cette crème est appliquée sur une peau psoriatique 1 à 2 fois par jour pendant 30 jours pour traitement d'attaque et indéfiniment pour entretien.
(f) On prépare un gel topique en procédant au mélange des ingrédients suivants :

| | |
|---|---|
| Composé de l'exemple 4 | 0,050 g |
| Ethanol | 43,000 g |
| α-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp. | 100,000 g |

Ce gel est appliqué dans le traitement de l'acné 1 à 3 fois par jour pendant 6 à 12 semaines selon la gravité du cas traité.
(g) On prépare une lotion capillaire ami-chute et pour la repousse des cheveux en procédant au mélange des ingrédients suivants :

| | |
|---|---|
| Composé de l'exemple 3 | 0,05 g |
| Composé vendu sous la dénomination "Minoxidil | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp. | 100,00 g |

On applique cette lotion 1 à 2 fois par jour pendant 3 mois sur un cuir chevelu ayant subi une chute de cheveu et indéfiniment pour traitement d'entretien.
(h) On prépare une crème anti-acnéique en procédant au mélange des ingrédients suivants :

| | |
|---|---|
| Composé de l'exemple 5 | 0,050 g |
| Acide rétinoïque | 0,010 g |
| Mélange de Stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2 130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp. | 100,000 g |

Cette crème est appliquée sur une peau atteinte de dermatose ou une peau acnéique 1 à 3 fois 10 par jour pendant 6 à 12 semaines.
(i) On prépare une crème huile dans l'eau en réalisant la formulation suivante :

| | |
|---|---|
| Composé de l'exemple 4 | 0,020 g |
| 17-valérate de bétamethasone | 0,050 g |
| S-carboxyméthyl cystéine | 3,000 g |
| St&rate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | 18,000 g |
| Perhydrosqualène | 4,000 g |
| Mélange de triglycérides caprylique-caprique vendu sous la dénomination de "Miglyol8 12" par la société "DYNAMIT NOBEL" Triéthanolamine (99 % en poids) | 2,500 g |
| Eau q.s.p. | 100,000 g |

Cette crème est appliquée 2 fois par jour sur une peau atteinte de dermatose inflammatoire pendant 30 jours.
(i) On prépare la crème de type huile dans l'eau suivante :

| | |
|---|---|
| Acide lactique | 5,000 g |
| Composé de l'exemple 1 | 0,020 g |
| St&rate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène 0,020 g | |
| Alcool cétostéarylique 6,200 g | |
| Conservateurs | q.s. |
| Perhydrosqualène | 18,000 g |
| Mélange dé triglycérides caprylique-caprique vendu sous la dénomination de "Miglyol 8 12" par la société "DYNAMIT NOBEL" | 4,000 g |
| Eau q.s.p | 100,000 g |

Cette crème est appliquée 1 fois par jour, elle aide à lutter contre le vieillissement qu'il soit photoinduit ou chronologique.
(k) On prépare l'onguent anhydre suivant :

| | |
|---|---|
| Composé de l'exemple 1 | 5,000 g |
| Huile de vaseline | 50,00 g |
| butylhydrotoluène | 0,050 g |
| vaseline blanche | qs 100 g |

Cet onguent est appliqué 2 fois par jour sur une peau atteinte de dermatose squameuse pendant 30 jours.

### 3) VOIE INTRALESIONNELLE

(a) On prépare la composition suivante :

| | |
|---|---|
| Composé de l'exemple 2 | 0,002 g |
| Oléate d'éthyle qs | 10 g |

Dans le traitement du mélanome malin, on injecte la composition à un individu adulte à une fréquence de 1 à 7 fois par semaine pendant 1 à 12 mois.
(b) On prépare la composition suivante :

| | |
|---|---|
| Composé de l'exemple 1 | 0,050 g |
| Huile d'olive | qs 2 g |

Dans le traitement du carcinome basocellulaire, on injecte la composition à un individu adulte à une fréquence de 1 à 7 fois par semaine pendant 1 à 12 mois.
(c) On prépare la composition suivante :

| | |
|---|---|
| Composé de l'exemple 3 | 0,1 mg |
| Huile de sésame | qs 2 g |

Dans le traitement du carcinome spinocellulaire, on injecte la composition à un individu adulte à une fréquence de 1 à 7 fois par semaine pendant 1 à 12 mois.
(d) On prépare la composition suivante :

| | |
|---|---|
| Composé de l'exemple 4 | 0,001 mg |
| Benzoate de méthyle | qs 10 g |

Dans le traitement du carcinome du colon, on injecte la composition à un individu adulte à une fréquence de 1 à 7 fois par semaine pendant 1 à 12 mois.

### 4) VOIE INTRAVEINEUSE

(a) On prépare l'émulsion lipidique injectable suivante :

| | |
|---|---|
| Composé de l'exemple 4 | 0,001 mg |
| Huile de soja | 10,000 g |
| Phospholipide d'oeuf | 1,200 g |
| Glycérine | 2,500 g |
| Eau pour injectable q.s.p. | 100,000 g |

Dans le traitement du psoriasis, on injecte la composition à un individu adulte à une fréquence de 1 à 7 fois par semaine pendant 1 à 12 mois.
(b) On prépare l'émulsion lipidique injectable suivante :

| | |
|---|---|
| Composé de l'exemple 1 | 0,010g |
| Huile de coton | 10,000 g |
| Lécithine de soja | 0,750 g |
| Sorbitol | 5,000 g |
| DL,α Tocophérol | 0,100 g |
| Eau pour injectable q.s.p. | 100,000 g |

Dans le traitement de l'ichtyose, on injecte la composition à un individu adulte à une fréquence de 1 à 7 fois par semaine pendant 1 à 12 mois.
(c) On prépare l'émulsion lipidique injectable suivante :

| | |
|---|---|
| Composé de l'exemple 2 | 0,001 g |
| Huile de soja | 15,000 g |
| Monoglycérides acétylés | 10,000 g |
| Pluronic F-108 | 1,000 g |
| Glycérol | 2,500 g |
| Eau pour injectable q.s.p. | 100,000 g |

Dans le traitement de la leucémie, on injecte la composition à un individu adulte à une fréquence de 1 à 7 fois par semaine pendant 1 à 12 mois.
(d) On prépare la composition micelle mixte suivante :

| | |
|---|---|
| Composé de l'exemple 2 | 0,001 g |
| Lécithine | 16,930 g |
| Acide glycocholique | 8,850 g |
| Eau pour injectable q.s.p. | 100,000 g |

Dans le traitement du mélanome malin, on injecte la composition à un individu adulte à une fréquence de 1 à 7 fois par semaine pendant 1 à 12 mois.
(e) On prépare la composition de cyclodextrine suivante :

| | |
|---|---|
| Composé de l'exemple 3 | 0,1 mg |
| beta-cyclodextrine | 0,100 g |
| Eau pour injectable q.s.p. | 10,000 g |

Dans le traitement du rejet de greffe, on injecte la composition à un individu adulte à une fréquence de 1 à 7 fois par semaine pendant 1 à 12 mois.
(f) On prépare la composition de cyclodextrine suivante :

| | |
|---|---|
| Composé de l'exemple | 0,010 g |
| 2-hydroxypropylcyclodextrine | 0,100 g |
| Eau pour injectable q.s.p. | 10,000 g |

Dans le traitement du cancer du rein, on injecte la composition à un individu adulte à une fréquence de 1 à 7 fois par semaine pendant 1 à 12 mois.

### EXEMPLE 7 : Exemple de test d'évaluation de l'activité biologiue des composés de l'invention : Potentiel de transactivation

L'activité agoniste VDR peut être testée sur la lignée cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après co-transfection, le produit test est ajouté dans le milieu. Après 18 heures de traitement, le dosage de l'activité CAT des lysats cellulaires est effectuée par un test Elisa (Enzyme Linked Immuno Sorbent Essay, test commercialisé par Roche Molecular Biochemicals). L'activité agoniste peut être caractérisée dans ce système de cotransfection, par la détermination de la dose nécessaire pour atteindre 50 % de l'activité maximale du produit (AC50).

**Tableau 1**

| **Composé** | **AC50 nM** |
|---|---|
| Exemple 1 | 39 |
| Exemple 59 de D1 | 124 |
| | |
| Exemple 2 | 77 |
| Exemple 80 de D1 | 746 |
| | |
| Exemple 3 | 7 |
| Exemple 92 de D1 | 79 |
| | |
| Exemple 4 | 16 |
| Exemple 80 de D1 | 746 |
| | |
| Exemple 5 | 3 |
| Exemple 60 de D1 | 319 |

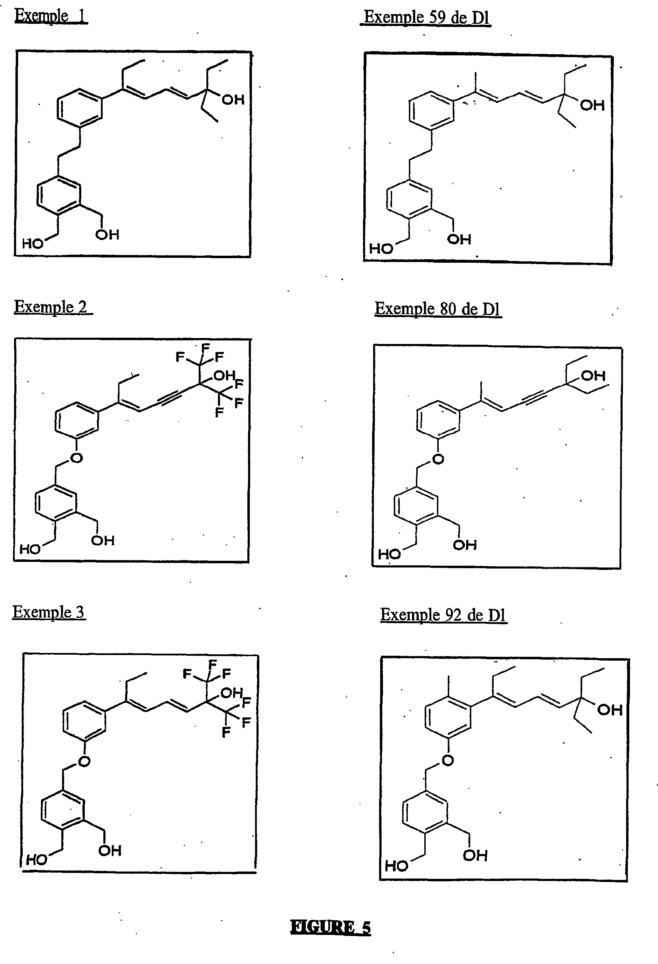

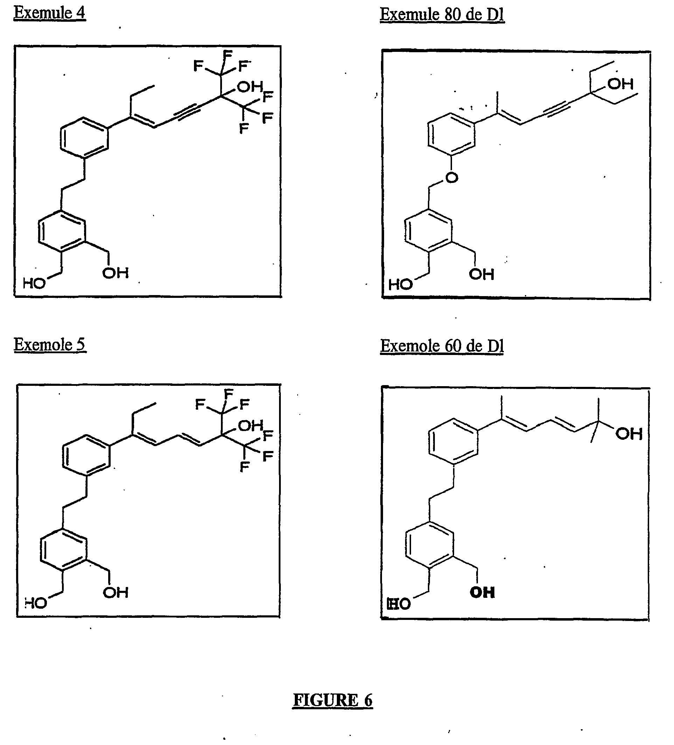

Chaque composé selon l'invention a été comparé au composé structurellement le plus proche protégé dans la demande de brevet WO 00/26167 (D1). Pour faciliter la comparaison, les structures des composés de l'invention et des exemples comparatifs de D1 sont regroupées sur les figures 5 et 6. Les résultats montrent l'activité significativement plus forte des composés de l'invention par rapport aux composés de D1. Une différence entre deux valeurs d'AC50 est considérée comme significative si elle est au moins d'un facteur 3, préférentiellement d'un facteur 5 et plus préférentiellement d'un facteur 10 .

### EXEMPLE 8 : Exemple de test d'évaluation de l'activité biologique des composés de l'invention : Activité sur la prolifération des kératinocytes humains

Il est connu que la 1,25-dihydroxyvitamine D3, appelée calcitriol et correspondant à la vitamine D naturelle inhibe la prolifération des kératinocytes humain en culture. La méthode utilisée est la suivante : les kératinocytes humains normaux sont ensemencés à basse densité dans une plaque 24 puits. Après 4 heures, les composés à tester sont ajoutés au milieu de culture. Après 5 jours de culture, la prolifération des kératinocytes est déterminée par incorporation de 5-bromo-2' deoxyuridine (BrdU) dans l'ADN. La quantité de BrdU incorporée est ensuite quantifiée en utilisant le test Elisa (Enzyme Linked Immuno Sorbent Essay, test commercialisé par Roche Molecular Biochemicals).

L'effet inhibiteur sur la prolifération des kératinocytes, des composés selon l'invention, et du calcitriol utilisé comme composé de référence, est résumé dans le tableau II suivant. La valeur Ic 50 indique la concentration en composé testé pour laquelle le composé inhibe de 50% la prolifération des kératinocytes.

Ces résultats permettent de mettre en évidence pour les composés selon l'invention une activité inhibitrice sur la prolifération des kératinocytes dans les mêmes gammes de valeur que celle du calcitriol (vitamine D naturelle). Par ailleurs les résultats montent des différences significatives entre les composés selon l'invention et les composés structurellement les plus proches de D1. Une différence est considérée comme significative entre deux valeurs d'AC50 si elle est au moins d'un facteur 3, préférentiellement d'un facteur 5 et plus préférentiellement d'un facteur 10.

**Tableau II**

| Composé | Inhibition dé la prolifération **IC 50* (nM)** |
|---|---|
| Calcitriol | 14 |
| Exemple 1 | 45 |
| Exemple 59 de D1 | 1029 |
| Exemple 2 | 153 |
| Exemple 80 de D1 | > 10000 (non actif) |
| | |
| Exemple 3 | 35 |
| Exemple 92 de D1 | 99 |
| | |
| Exemple 4 | 29 |
| Exemple 80 de D 1 > 10000 (non actif) | |
| | |
| Exemple 5 | 8 |
| Exemple 60 de D1 | 1506 |

| | |
|---|---|
| * Concentration pour laquelle on obtient 50 % d'inhibition de la prolifération des kératynocytes. | |

### EXEMPLE 9 : Exemple de test d'évaluation de l'activité biologique des composés de l'invention : Activité sur la différentiation des cellules HL60.

Le Calcitriol induit la différentiation des cellules de leucémie promyélocytaire (HL60) en monocytes/macrophages. Cet effet inducteur de différentiation est un marqueur bien caractérisé de l'activité Vitamine D cellulaire. Un des produits antimicrobien le plus important des macrophages, est le peroxyde d'hydrogène, qui peut être analysé expérimentalement par la réduction du NBT (Nitroblue Tetrazolium).

La méthode utilisée est la suivante : Les cellules HL60 sont ensemencées dans des plaques 6 puits puis traitées immédiatement avec les composés à tester. Après 4 jours de culture, les cellules sont incubées avec du TPA ester de phorbol et le NBT pendant une courte période et les cellules différentiées, c.a.d, positives au NBT, sont comptabilisées.

L'effet inducteur de la différentiation sur les cellules HL60, des composés selon l'invention ainsi que celui du composé de référence le calcitriol est explicité dans le tableau III ci-dessous.

**Tableau III**

| Composé | Activation de la différentiation |
|---|---|
| | AC 50" (nM) |
| Calcitriol | 7 (n =5) |
| Exemple 1 | 56 (n = 3) |
| Exemple 59 de D1 | 310 |
| Exemple 3 | 7 (n = 2) |
| Exemple 5 | 5 (n = 2) |

| | |
|---|---|
| * Concentration pour laquelle on obtient 50% de la réponse maximale d'activation de la différenciation. | |

Ces résultats montrent que les exemples 3 et 5 selon l'invention présentent une activité d'induction de la différentiation sur les cellules HL60 similaire à celle de Calcitriol.

### EXEMPLE 10 : Exemple de test d'évaluation de l'activité biologigue des composés de l'invention : Induction "in vivo" de la 24-Hydroxylase chez la souris SKH

La 24-Hydroxylase est un enzyme cytochrome P450 qui hydroxyle la 1,25-dihydroxyvitamine D3 (Calcitriol) en position 24 résultant en un métabolite la la, 24, 25-trihydroxyvitamin D3. Il a été montré par Voorhees and al. (JID 1997.108 : 513-518) que l'expression du gêne de la 24-Hydroxylase était induit par le calcitriol dans la peau humaine.

Par conséquent, l'activité agoniste aux récepteurs de la vitamine D des composés de l'invention peut être évaluée "in vivo" par induction de la 24-Hydroxylase chez la souris SKH.

La méthode utilisée est la suivante : Des souris XII reçoivent une application topique unique d'un composé à tester en solution dans l'éthanol à des concentrations croissantes.

Un volume de 50 µl du produit à tester ou du véhicule seul est appliqué sur le dos des souris à l'aide d'une pipette.

D'autres souris SKH reçoivent une application topique unique de Calcitriol en solution dans l'éthanol à des concentrations croissantes. Un volume de 50 µl du produit à tester ou du véhicule seul est appliqué sur le dos des souris à l'aide d'une pipette.

8 heures après l'application topique, les souris sont euthanasiées, la peau traitée est prélevée et l'épiderme est séparé du derme. La quantification de l'ARNm de la 24-Hydroxylase est réalisée par PCR semi quantitative. Les résultats sont normalisés par rapport à l'expression de l'ARNm de GAPDH et les valeurs pour les différentes concentrations de Calcitriol testées et, pour les différents composés de l'invention testés, sont exprimés en facteur d'induction par rapport au Calcitriol.

Les résultats sont résumés dans le tableau IV suivant:

**Tableau IV : Expression de l'ARNm de la 24-Hydroxylase**

| **Composé testé** | **Concentration % (poids/volume)** | **Facteur d'inductino Versus éthanol** |
|---|---|---|
| Calcitriol | 0.0001 | 6.7 |
| Calcitriol | 0.001 | 10.3 |
| Calcitriol | 0.01 | 20.1 |
| Calcitriol | 0.1 | 26 |

Ces résultats montrent que le Calcitriol administré en application topique unique induit chez la souris de façon dose-dépendante l'expression de l'ARNm de la 24-hydroxylase dans l'épiderme.

L'activité biologique des composés de l'invention est évaluée par comparaison entre les facteurs d'inductions obtenus pour les composés de l'invention et les facteurs d'inductions obtenus pour le Calcitriol. Les résultats sont présentés dans le tableau V suivant :

**Tableau V**

| **Composé testé** | **Concentration % (poids/volume)** | **Induction en % vs Induction calcitriol Testé à 0.01 %** |
|---|---|---|
| Calcitriol | 0.01 | 100 |
| Exemple 1 | 0.1 | 108 |
| Exemple 2 | 0.01 | 58 |
| Exemple 3 | 0.001 | 59 |
| Exemple 4 | 0.01 | 89 |
| Exemple 5 | 0.0003 | 106 |

Ainsi, ces résultats montrent que les exemples selon l'invention, présentent une activité comparable ou très supérieure (exemple 3 et 5) à celle du Calcitriol.

## Revendications

1. Composés **caractérisés par le fait qu'**ils sont pris, seuls ou en mélanges, dans le groupe constitué par :
- (4E,6E)-7-{3-[2-(3,4-Bis-hydroxymethyl-phenyl)-ethyl]-phenyl}-3-ethyl-nona-4,6-dien-3-ol,
- (E)-6-[3-(3,4-Bis-hydroxymethyl-benzyloxy)-phenyl]-1,1,1-trifluoro-2-trifluoromethyl-oct-5-en-3-yn-2-ol,
- (3E,5E)-6-[3-(3,4-Bis-hydroxymethyl-benzyloxy)-phenyl]-1,1,1-trifluoro-2-trifluoromethyl-octa-3,5-dien-2-ol,
- (E)-6-{3-[2-(3,4-Bis-hydroxymethyl-phenyl)-ethyl]-phenyl}-1,1,1-trifluoro-2-trifluoromethyl-oct-5-en-3-yn-2-ol,
- (3E,5E)-6-{3-[2-(3,4-Bis-hydroxymethyl-phenyl)-ethyl]-phenyl-1,1,1-trifluoro-2-trifluoromethyl-octa-3,5-dien-2-ol,
ainsi que leurs isomères géométriques et les composés pour lesquels une ou plusieurs des fonctions hydroxyles sont protégées par un groupement protecteur de type -(C=O)-R, avec R représentant un radical alkyle linéaire ou ramifié ayant de 1 à 6 atomes de carbone, ou un radical aryle ayant de 6 à 10 atomes de carbone, ou un radical aralkyle ayant de 7 à 11 atomes de carbone, le radical aryle ou le radical aralkyle pouvant être mono ou disubstitué par un groupement hydroxy, par un groupement alkoxy ayant de 1 à 3 atomes de carbone, par un atome d'hahogène, par une fonction nitro ou par une fonction amino.

2. Composés selon la revendication 1, **caractérisés en ce que** le radical alkyle linéaire ou ramifié ayant de 1 à 6 atomes de carbone est choisi parmi un radical méthyle, éthyle, isopropyle, tertiobutyle ou hexyle.

3. Composés selon la revendication 1, **caractérisés en ce que** le radical aryle ayant de 6 à 10 atomes de carbone est choisi parmi un radical phényle ou naphtyle.

4. Composés selon la revendication 1, **caractérisés en ce que** le radical aralkyle ayant de 7 à 11 atomes de carbone est choisi parmi un radical benzyle ou méthyl-naphtyle.

5. Composés selon la revendication 1, **caractérisés en ce que** l'atome d'halogène est choisi parmi un atome de fluor, de brome ou de chlore.

6. Composés selon l'une quelconque des revendications 1 à 5 à titre de médicament.

7. Utilisation d'un composé selon l'une quelconque des revendications 1 à 5 pour la fabrication d'un médicament destiné au traitement :
- des affections dermatologiques liées à un désordre de la kératinisation portant sur la différenciation et sur la prolifération telles que les acnés vulgaires, comédoniennes, polymorphes, rosacées, les acnés nodulokystiques, conglobata, les acnés séniles, les acnés secondaires telles que l'acné solaire, médicamenteuse ou professionnelle ; des ichtyoses, des états ichtyosiformes, de la maladie de Darrier, des kératodermies palmoplantaires, des leucoplasies et des états leucoplasiformes, du lichen cutané ou muqueux (buccal) ;
- des affections dermatologiques avec une composante immuno-allergique inflammatoire, avec ou sans trouble de la prolifération cellulaire, telles que toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
- des proliférations dermiques ou épidermiques qu'elles soient bénignes ou malignes, qu'elles soient ou non d'origine virale telles que verrues vulgaires, les verrues planes et l'épidermodysplasie verruciforme, les papillômatoses orales ou florides, le lymphome T, et les proliférations pouvant être induites par les ultraviolets notamment dans le cas des épithélioma baso et spinocellulaires, ainsi que les lésions précancéreuses cutanées telles que les kératoacanthomes;
- des dermatoses immunes telles le lupus érythémateux, les maladies immunes bulleuses ou les maladies du collagène, telle la sclérodermie;
- des affections dermatologiques ou générales à composante immunologique;
- des troubles de la fonction sébacée tels que l'hyperséborrhée de l'acné ou la séborrhée simple;
- des désordres cutanés dus à une exposition aux rayonnements U.V., du vieillissement de la peau, qu'il soit photo-induit ou chronologique, des pigmentations et les kératoses actiniques, ou toutes pathologies associées au vieillissement chronologique ou actinique;
- des troubles de la cicatrisation ou des vergetures,
- des affections inflammatoires telles que l'arthrite, des affections d'origine virale au niveau cutané ou général, telle que le syndrome de Kaposis;
- des troubles ophtalmologiques, notamment les cornéopathies;
- des états cancéreux ou précancéreux de cancers présentant ou pouvant être induits par des récepteurs de vitamine D, tels que le cancer du sein, la leucémie, les syndromes myélodysplasiques et les lymphomes, les carcinomes des cellules de l'épithélium malpighien et les cancers gastro-intestinaux, les mélanomes, et l'ostéosarcome ;
- de l'alopécie de différentes origines, notamment l'alopécie due à la chimiothérapie ou aux rayonnements;
- des affections immunitaires, telles que les maladies auto-immunes, le diabète sucré de type 1, la sclérose en plaques, le lupus et les affections de type lupus, l'asthme, la glomérulonéphrite; des dysfonctionnements sélectifs du système immunitaire, telles que le SIDA, du rejet immun; - des affections endocriniennes ;
- des affections **caractérisées par** une gestion anormale du calcium intracellulaire, des pathologies dans lesquelles le métabolisme calcique est impliqué, telle que l'ischémie musculaire;
- des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, ou les troubles de la fonction parathyroïdienne;
- des affections du système cardiovasculaire telles que l'artériosclérose ou l'hypertension ainsi que le diabète non-insulino dépendant.

8. Composition pharmaceutique, **caractérisée par le fait qu'**elle comprend, dans un support pharmaceutiquement acceptable, au moins l'un des composés tels que définis à l'une quelconque des revendications 1 à 5.

9. Composition selon la revendication 8, **caractérisée en ce que** la concentration en composé(s) selon la revendication 1 est comprise entre 0,001 % et 5 % en poids par rapport au poids total de la composition.

10. Composition cosmétique, **caractérisée par le fait qu'**elle comprend, dans un support cosmétiquement acceptable, au moins l'un des composés tels que définis à l'une quelconque des revendications 1 à 5.

11. Composition selon la revendication 10, **caractérisée en ce que** la concentration en composé(s) est comprise entre 0,001 % et 3 % en poids par rapport au poids total de la composition.

12. Utilisation cosmétique d'un composé tel que défini à l'une des revendications 1 à 5 en tant qu'agent pour l'hygiène corporelle ou capillaire.

## Claims

1. Compounds **characterized in that** they are taken, alone or in a mixture, from the group formed by:
- (4E,6E)-7-{3-[2-(3,4-bishydroxymethylphenyl)-ethyl]phenyl}-3-ethylnona-4,6-dien-3-ol,
- (E)-6-[3-(3,4-bishydroxymethylbenzyloxy)phenyl]-1,1,1-trifluoro-2-trifluoromethyloct-5-en-3-yn-2-ol,
- (3E,5E)-6-[3-(3,4-bishydroxymethylbenzyloxy)-phenyl]-1,1,1-trifluoro-2-trifluoromethylocta-3,5-dien-2-ol,
- (E)-6-{3-[2-(3,4-bishydroxymethylphenyl)ethyl]-phenyl}-1,1,1-trifluoro-2-trifluoromethyloct-5-en-3-yn-2-ol,
- (3E,5E)-6-{3-[2-(3,4-bishydroxymethylphenyl)-ethyl]phenyl-1,1,1-trifluoro-2-trifluoromethylocta-3,5-dien-2-ol,
as well as their geometric isomers and the compounds for which one or more of the hydroxyl functions are protected by a protective group of type -(C=O)-R,
with R representing a linear or branched alkyl radical having from 1 to 6 carbon atoms, or an aryl radical having from 6 to 10 carbon atoms, or an aralkyl radical having from 7 to 11 carbon atoms, the aryl radical or the aralkyl radical being able to be mono- or disubstituted by a hydroxy group, by an alkoxy group having from 1 to 3 carbon atoms, by a halogen atom, by a nitro function or by an amino function.

2. Compounds according to Claim 1, **characterized in that** the linear or branched alkyl radical having from 1 to 6 carbon atoms is chosen from a methyl, ethyl, isopropyl, tert-butyl or hexyl radical.

3. Compounds according to Claim 1, **characterized in that** the aryl radical having from 6 to 10 carbon atoms is chosen from a phenyl or naphthyl radical.

4. Compounds according to Claim 1, **characterized in that** the aralkyl radical having from 7 to 11 carbon atoms is chosen from a benzyl or methylnaphthyl radical.

5. Compounds according to Claim 1, **characterized in that** the halogen atom is chosen from a fluorine, bromine or chlorine atom.

6. Compounds according to any one of Claims 1 to 5 as a medicament.

7. Use of a compound according to any one of Claims 1 to 5 for the production of a medicament for use in the treatment:
- of dermatological complaints linked to a keratinization disorder having a bearing on differentiation and proliferation such as common acne, blackheads, polymorphs, rosacea, nodulocystic acne, acne conglobata, senile acne, secondary acne such as solar, medicinal or professional acne; ichthyosis, ichthyosiform states, Darrier's syndrome, palmoplantar keratodermia, leucoplasia and leucoplasiform states, cutaneous or mucous (buccal) lichen;
- dermatological complaints with an inflammatory immunoallergic component, with or without cell proliferation disorder, such as all forms of psoriasis, whether it be cutaneous, mucous or ungual, and even psoriatic rheumatism, or else cutaneous atopy, such as eczema or respiratory atopy or else gingival hypertrophy;
- dermal or epidermal proliferations whether they are benign or malignant, whether they are or are not of viral origin such as common warts, planar warts and verruciform epidermodysplasia, oral or florid papillomatoses, T lymphoma, and proliferations being able to be induced by ultraviolet, especially in the case of baso- and spinocellular epithelioma, as well as cutaneous precancerous lesions such as keratoacanthomas;
- immune dermatoses such as lupus erythematosus, bullous immune diseases or collagen diseases, such as scleroderma;
- dermatological or general complaints with an immunological component;
- sebaceous function disorders such as hyperseborrhea of acne or simple seborrhea;
- cutaneous disorders due to exposure to UV rays, ageing of the skin, whether it be photoinduced or chronological, pigmentations and actinic keratoses, or any pathologies associated with chronological or actinic ageing;
- cicatrization disorders or stretch marks,
- inflammatory complaints such as arthritis, complaints of viral origin at the cutaneous or general level, such as Kaposi's syndrome;
- ophthalmological complaints, especially corneopathy;
- cancerous or precancerous states of cancers having or being able to be induced by vitamin D receptors, such as breast cancer, leukemia, myelodysplasic syndromes and lymphomas, carcinomas of the cells of the malpighian epithelium and gastrointestinal cancers, melanomas, and osteosarcoma;
- alopecia of different origins, especially alopecia due to chemotherapy or to radiation;
- immune complaints, such as autoimmune diseases, diabetes mellitus of type 1, multiple sclerosis, lupus and lupus type complaints, asthma, glomerulonephritis; selective dysfunctions of the immune system, such as AIDS, immune rejection; endocrine complaints;
- complaints **characterized by** an abnormal management of intracellular calcium, pathologies in which the calcium metabolism is involved, such as muscular ischemia;
- deficiencies in vitamin D and other complaints of the homeostasis of minerals in the plasma and the bones, such as rachitis, osteomalacia, osteoporosis, especially in the case of menopausal women, renal osteodystrophia, or complaints of the parathyroid function;
- complaints of the cardiovascular system such as arteriosclerosis or hypertension as well as non-insulin-dependent diabetes.

8. Pharmaceutical composition, **characterized in that** it comprises, in a pharmaceutically acceptable support, at least one of the compounds such as defined in any one of Claims 1 to 5.

9. Composition according to Claim 8, **characterized in that** the concentration of compound(s) according to Claim 1 is between 0.001% and 5% by weight with respect to the total weight of the composition.

10. Cosmetic composition, **characterized in that** it comprises, in a cosmetically acceptable support, at least one of the compounds such as defined in any one of Claims 1 to 5.

11. Composition according to Claim 10, **characterized in that** the concentration of compound(s) is between 0.001% and 3% by weight with respect to the total weight of the composition.

12. Cosmetic use of a compound such as defined in one of Claims 1 to 5 as an agent for body or hair hygiene.

## Patentansprüche

1. Verbindungen, **dadurch gekennzeichnet, dass** sie einzeln oder in Form von Gemischen aus der folgenden Gruppe gewählt sind, bestehend aus:
- (4E, 6E)-7-{3-[2-(3,4-Bis-hydroxymethyl-phenyl)-ethyl]-phenyl}-3-ethyl-nona-4,6-dien-3-ol,
- (E)-6-[3-(3,4-Bis-hydroxymethyl-benzyloxy)-phenyl]-1,1,1-trifluor-2-trifluormethyl-oct-5-en-3-in-2-ol,
- (3E,5E)-6-[3-(3,4-Bis-hydroxymethyl-benzyloxy)-phenyl]-1, 1, 1-trifluor-2trifluormethyl-octa-3,5-dien-2-ol,
- (E)-6-{3-[2-(3,4-Bis-hydroxymethyl-phenyl)-ethyl]-phenyl}-1, 1,1-trifluor-2-trifluormethyl-oct-5-en-3-in-2-ol,
- (3E,5E)-6-{3-[2-(3,4-Bis-hydroxymethyl-phenyl)-ethyl]-phenyl-1,1,1-trifluor-2-trifluormethyl-octa-3,5-dien-2-ol,
sowie ihre geometrischen Isomere und Verbindungen, bei denen eine oder mehrere Hydroxyfunktionen durch eine Schutzgruppe vom Typ -(C=O)-R geschützt sind, wobei R eine geradkettige oder verzweigte Alkylgruppe mit 1 bis 6 Kohlenstoffatomen oder eine Arylgruppe mit 6 bis 10 Kohlenstoffatomen oder eine Aralkylgruppe mit 7 bis 11 Kohlenstoffatomen bedeutet, wobei die Arylgruppe oder die Aralkylgruppe mit einer Hydroxygruppe, mit einer Alkoxygruppe mit 1 bis 3 Kohlenstoffatomen, mit einem Halogenatom, mit einer Nitrofunktion oder mit einer Aminofunktion ein oder mehrfach substituiert sein können.

2. Verbindungen nach Anspruch 1, **dadurch gekennzeichnet, dass** die geradkettige oder verzweigte Alkylgruppe mit 1 bis 6 Kohlenstoffatomen unter Methyl, Ethyl, Isopropyl, *tert*-Butyl oder Hexyl ausgewählt ist.

3. Verbindungen nach Anspruch 1 **dadurch gekennzeichnet, dass** die Arylgruppe mit 6 bis 10 Kohlenstoffatomen unter Phenyl oder Naphthyl ausgewählt ist.

4. Verbindungen nach Anspruch 1, **dadurch gekennzeichnet, dass** die Aralkylgruppe mit 7 bis 11 Kohlenstoffatomen unter Benzyl oder Methylnaphthyl ausgewählt ist.

5. Verbindungen nach Anspruch 1, **dadurch gekennzeichnet, dass** das Halogenatom unter Fluor, Brom oder Chlor ausgewählt ist.

6. Verbindungen nach einem der Ansprüche 1 bis 5 als Arzneimittel.

7. Verwendung einer Verbindung nach einem der Ansprüche 1 bis 6 für die Herstellung eines Arzneimittels, das vorgesehen ist für die Behandlung von:
- dermatologischen Erkrankungen, die mit einer Verhornungsstörung verbunden sind, die auf der Differenzierung und Proliferation der Zellen beruht, wie Acne vulgaris, Acne comedonica, polymorpher Akne, Acne rosaceae, nodulocystischer Akne, Acne conglobata, Acne senilis und sekundären Akneformen, wie Acne solaris, Acne medicamentosa oder Acne professionalis; Ichthyosis, ichtyosisartigen Zuständen, der Darier Krankheit, Palmoplantarkeratosen, Leukoplakien und leucoplakieformen Zuständen und Lichen der Haut oder der Schleimhäute (buccal);
- dermatologischen Erkrankungen mit entzündlicher immunoallergischer Komponente, mit oder ohne einer Störung der Proliferation der Zellen, wie aller Arten von Psoriasis der Haut, der Schleimhäute oder der Nägel, Psoriasis arthropathica, oder auch Atopie der Haut, wie Ekzemen, Atopie der Atemwege oder Hypertrophie des Zahnfleisches;
- Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida, T-Lymphom und Proliferationen, die von UV-Strahlung induziert sein können, insbesondere im Falle von Epithelioma basocellulare und spinocellulare, sowie alle präkanzerösen Hautläsionen, wie Keratoacanthomen;
- Immundermatosen, beispielsweise Lupus erythematodes, bullösen Immunerkrankungen und Kollagenerkrankungen, wie Sklerodermie;
- dermatologischen oder allgemeinen Erkrankungen mit immunologischer Komponente;
- Störungen der Talgdrüsenfunktion, wie Hyperseborrhoe bei Akne oder Seborrhoe simplex;
- Hautstörungen, die mit einer UV-Exposition zusammenhängen; altersbedingter oder lichtinduzierter Hautalterung, aktinischen Pigmentierungen und Keratosen, oder allen Pathologien, die mit der altersbedingten oder aktinischen Hautalterung einhergehen;
- Störungen der Wundheilung oder Streifen;
- entzündlichen Erkrankungen, wie Arthritis, Erkrankungen viraler Herkunft auf dem Niveau der Haut oder in generalisierter Form, wie zur Behandlung des Kaposi-Syndroms;
- ophthalmologischen Störungen, wie Corneopathien;
- kanzerösen oder präkanzerösen Zuständen, bei denen Vitamin D-Rezeptoren vorliegen oder bei denen Vitamin D-Rezeptoren induziert werden können, wie beispielsweise Brustkrebs, Leukämie, myelodysplastischen Syndromen und Lymphomen, Karzinomen der Zellen des Mapighie-Epithels und gastrointestinalen Karzinomen, Melanomen und Osteosarkom;
- Alopezie unterschiedlicher Herkunft, insbesondere durch Chemotherapie oder UV-Strahlung verursachter Alopezie;
- Immunerkrankungen, wie Autoimmunkrankheiten, Diabetes vom Typ 1, Multiple Sklerose, Lupus und Erkrankungen vom Lupus-Typ, Asthma, Glomerulonephritis, selektiven Funktionsstörungen des Immunsystems, beispielsweise AIDS, Immunabstoßung;
- endokrinen Erkrankungen;
- Erkrankungen, die durch ein anormales Management des intrazellulären Calciums charakterisiert sind, Erkrankungen, bei denen der Calcium-Stoffwechsel beteiligt ist, wie muskuläre Ischämie;
- Vitamin D-Mangel und weiteren Erkrankungen der Homöostase von Mineralstoffen im Plasma und in den Knochen, wie Rachitis, Osteomalazie, Osteoporose, insbesondere bei Frauen in den Wechseljahren, renale Osteodystrophie, Störungen der Nebenschilddrüsenfunktion.;
- Erkrankungen des kardiovaskulären Systems, wie Arteriosklerose oder Bluthochdruck sowie nichtinsulinabhängigem Diabetes.

8. Pharmazeutische Zusammensetzung, **dadurch gekennzeichnet, dass** sie in einem physiologisch akzeptablen Trägerstoff mindestens eine Verbindung nach einem der Ansprüche 1 bis 5 enthält.

9. Zusammensetzung nach Anspruch 8, **dadurch gekennzeichnet, dass** die Konzentration der Verbindung(en) nach Anspruch 1 im Bereich von 0,001 bis 5 Gew.-%, bezogen auf das Gesamtgewicht der Zusammensetzung, liegt.

10. Kosmetische Zusammensetzung, **dadurch gekennzeichnet, dass** sie in einem kosmetisch akzeptablen Träger mindestens eine Verbindung nach einem der Ansprüche 1 bis 5 enthält.

11. Zusammensetzung nach Anspruch 10, **dadurch gekennzeichnet, dass** die Konzentrationen der Verbindung(en) im Bereich von 0,001 bis 3 Gew.-%, bezogen auf das Gesamtgewicht der Zusammensetzung, liegt.

12. Kosmetische Verwendung einer Zusammensetzung nach einem der Ansprüche 1 bis 5 als Mittel für die Körper- oder Haarpflege.
